Europäisches Patentamt

European Patent Office

Office européen des brevets

(11) Publication number: **0 406 811 A2**

## EUROPEAN PATENT APPLICATION

(21) Application number: 90112702.7

(22) Date of filing: 03.07.90

(51) Int. Cl.⁵: **A61K 47/48, A61K 31/19**

(30) Priority: 03.07.89 JP 171551/89
08.06.90 JP 151292/90

(43) Date of publication of application:
09.01.91 Bulletin 91/02

(84) Designated Contracting States:
**BE DE ES FR GB IT**

(71) Applicant: AJINOMOTO CO., INC.
5-8, Kyobashi 1-chome, Chuo-ku
Tokyo 104(JP)

(72) Inventor: Tanabe, Toshiya, c/o Central
Research Laboratories
Ajinomoto Co., Inc., No. 1-1, Suzuki-cho
Kawasuki-ku, Kawasaki-shi,
Kanagawa-ken(JP)
Inventor: Kishimoto, Shin'ichi, c/o Central
Research Lab.
Ajinomoto Co., Inc., No. 1-1, Suzuki-cho
Kawasuki-ku, Kawasaki-shi,
Kanagawa-ken(JP)
Inventor: Yukawa, Toshihide, c/o Central
Research Lab.
Ajinomoto Co., Inc., No. 1-1, Suzuki-cho
Kawasuki-ku, Kawasaki-shi,
Kanagawa-ken(JP)

(74) Representative: Strehl, Schübel-Hopf,
Groening
Maximilianstrasse 54 Postfach 22 14 55
D-8000 München 22(DE)

(54) Novel clathrate compounds and a drug comprising them.

(57) A clathrate compound comprising an α-keto-acid and a cyclodextrin, useful as a drug for the treatment and prevention of renal disorders.

# NOVEL CLATHRATE COMPOUNDS AND A DRUG COMPRISING THEM

Technical Field

This invention relates to novel clathrate compounds comprising an $\alpha$-keto-acid and a cyclodextrin which are useful as nutrients, as medicines and as amino acid precursors.

Prior Art

It is known that $\alpha$-keto-acids, particularly many of those which are precursors of essential and quasi-essential amino acids, deeply participate in the metabolism of living bodies and are hence effective for the nourishment, treatment and prevention for patients suffering from renal disorders (uraemia, etc.), hepatic diseases (excess of ammonia, portal encephalopathies, etc.), and other protein- and nitrogen-wasting diseases (cf. U.S. Patent No. 4,100,293 and NO. 4,100,160; Japanese Patent Publication No. 38421/1983 and No. 24094/1985).

However, the problem associated with the administration of such an $\alpha$-keto-acid (hereinafter simply abbreviated as "a keto-acid") to the patients suffering from these diseases is its disagreeable odor and taste as well as its lacking stability. The keto-acid may be used as free acid, sodium salt, calcium salt, etc. However, any of these forms is not free of the above-mentioned problem. The free acid is poor in stability, and is liquid in most cases and hence is not easy to handle. The sodium and calcium salts are stable powders, but are also undesirable, since the sodium salt adversely affects the patients suffering from renal diseases, and in the case of calcium salt, much water has to be administered because of its low solubility.

Use of salts of keto-acids with basic amino acids disclosed in Japanese Patent Publication no. 38421/1983 and No. 24094/1985 seems to partially solve the above-mentioned problems. That is, the salts of keto-acids with basic amino acids obtained in the form of powders having a very high solubility in water and exhibiting slightly reduced disagreeable odor and taste. However, the stability remains still unsatisfactory, and the compounds undergo marked degradation upon storage at room temperature over several months. Particularly when an optically active keto-acid (e.g., $\alpha$-keto-$\beta$-methyl valeric acid, which is the keto analogue of isoleucine) is used, it is practically almost difficult to maintain its optical purity (J. Biol. Chem., 190 , 269 (1951).

Problems to be solved by the Invention

It has been desired to develop a method for improving $\alpha$-keto-acids in terms of odor, taste and stability.

Means to solve the Problems

Intensive studies to solve the above-mentioned problems have led the present inventors to find that keto-acids can form clathrate compounds with cyclodextrin under acidic conditions and that the clathrate compounds thus formed are white powders which are substantially odorless, have improved taste and are stable, including optical purity. This invention has been accomplished on the basis of these findings.

That is, this invention relates to novel clathrate compounds comprising an $\alpha$-keto-acid and a cyclodextrin.

The cyclodextrin used in this invention includes not only non-branched cyclodextrin in which six, seven or eight glucose molecules are combined through $\alpha$-1,4-linkage, which is commonly called $\alpha$-, $\beta$- or $\gamma$-cyclodextrin, but also macrocyclic cyclodextrin such as ô- or other cyclodextrin composed of nine or more glucose molecules and modified derivatives thereof. For example, methylated cyclodextrin in which a part of the hydroxy groups in each glucose molecule constituting cyclodextrin have ben methylated, and branched cyclodextrin havng straight or branched malto-oligosaccharides such as glucose, maltose, panose, etc., attached to the cyclodextrin ring (refer to Japanese Patent Kokai No. 106595/1979, No. 197602/1986 and No. 287901/1986) , are suitable for the present invention. The clathrate compounds of this invention comprising such cyclodextrin derivatives are more advantageous in terms of solubility in water than clathrate compounds comprising ordinary cyclodextrin. Many of these cyclodextrin derivates are natural additives, which are approved in Japan as non-official medicinal additives harmless to human bodies.

$\alpha$-Keto-acids (RCOCOOH) can be prepared by direct synthesis (refer to Japanese Patent Kokai No. 46920/1978) or by enzymatic oxidation of amino acids (Japanese Patent Publication No. 44715/1981, etc.). Keto-acids which can be used in this invention are not particularly limited. For example, the residue R is an organic residue selected from the group consisting of straight chain or branched aliphatic residues which are optionally substituted by amino groups, hydroxy groups, carboxyl groups, a further keto group, thiol groups, a halogen atom, such as a chlorine atom, an aromatic group, a heterocyclic group or which may comprise a carboxamide ester group or an oxygen atom, or R is a residue containing a steroid or non-aromatic ring compound or a side chain containing a heterocyclic ring.

Preferably, the residue R comprises 1 to 15 carbon atoms. Approximately 200 kinds of compounds are now known as keto-acids. Studying sufficient numbers of these keto-acids including keto analogues of essential and quasi-essential amino acids, the present inventors have confirmed that the above-mentioned properties can be markedly improved in any of these keto-acids. Representative examples of keto-acids include pyruvic acid, α-keto-isovaleric acid, α-keto-isocaproic acid, α-keto-β-methylvaleric acid, indolepyruvic acid, phenylpyruvic acid, α-keto-γ-methyolbutyric acid, imidazolepyruvic acid, etc. However, the keto-acids are not limited to these compounds.

The clathrate compounds of this invention consist of a keto-acid generally called "guest molecule" and cyclodextrin or a derivative thereof called "host molecule". The molar ratio of the guest molecule to the host molecule is generally in the range of 0.1:1 to 10:1, more preferably, in the range of 0.5:1 to 5:1, from viewpoints of dosage of keto-acids and prevention of disagreeable odor.

The clathrate compounds of the present invention may be prepared by any known method, e.g. by the method of preparation in saturated solution, the kneading method and the like (Nobuyuki Nakamura and Koki Horikoshi; Fragrance Journal, No. 63, 50 (1983)).

The thus prepared clathrate compounds of the present invention are substantially odorless white powders, as compared to keto-acids or their salts alone. In adidtion, no decompositon is noted and the compounds are stable even at room temperature. In particular, the clathrate compounds of keto-acids having optical activity are stable without substantially decreasing their optical purity. For example, the clathrate compound of optically active S-α-keto-β-methylvaleric acid obtained by enzymatic oxidative deamination and β-cyclodextrin causes no reduction in optical purity even after allowing to stand at normal temperature for 4 months.

Furthermore, the clathrate compounds of the present invention may be applied, if necessary, in the form of tablets, powders, capsules, sugar-coated tablets, solutions, etc. This is, as tablets, capsules, etc., binders such as traganth, gum arabic, corn starch or gelatin; an excipient such as microcrystalline cellulose; a swelling agent such as corn starch, ungelatinized starch, alginic acid, etc., a lubricating agent such as magnesium stearate; a sweetener such as sucrose, lactose and saccharin; and flavoring essence such as peppermint oil and cherry; may be used as components. Tablets may be coated with shellac and/or sugar. Solutions may also contain a sweetener such as sucrose, a preservative such as methylparaben and propylparaben, a colorant and flavor essence such as flavor essence or cherry and orange.

Furthermore, if required, a buffering agent and an antioxidant may also be included in the formulation.

The clathrate compounds of this invention show acidity of pH of 1 to 3, when dissolved in water; hence, a neutralizer such as basic substances such as basic amino acids, inorganic bases and the like may also be added when required.


Brief Description of the drawings

Figure 1 shows the powder X-ray diffraction pattern of the KMV/β-CD clathrate compound obtained in Example 1.

Figure 2 shows the powder X-ray diffraction pattern of the KIC/γ-CD clathrate compound obtained in Example 2.

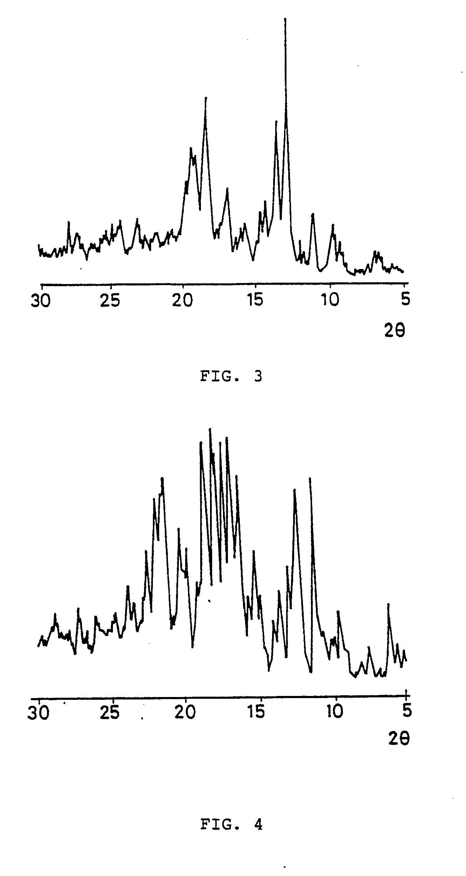

Figure 3 and Figure 4 show the powder X-ray diffraction pattern of cyclodextrins used in this invention (β-CD and γ-CD, respectively).

Figure 5 shows the solid NMR chart of PPA/β-CD clathrate compound obtained in Example 3 of this invention (measurement condition: 4.4 KHz).

Figure 6 shows the solid NMR chart of PPA used in this invention.

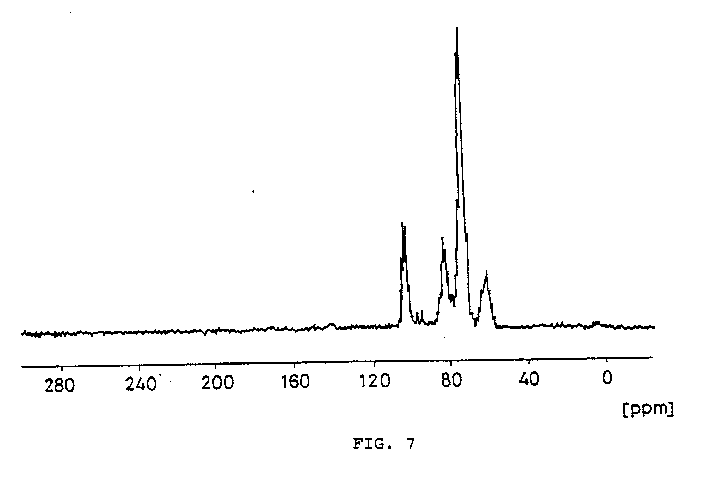

Figure 7 shows the solid NMR chart of β-CD used in this invention.

Hereafter the present invention is described by referring to the examples.


Example 1

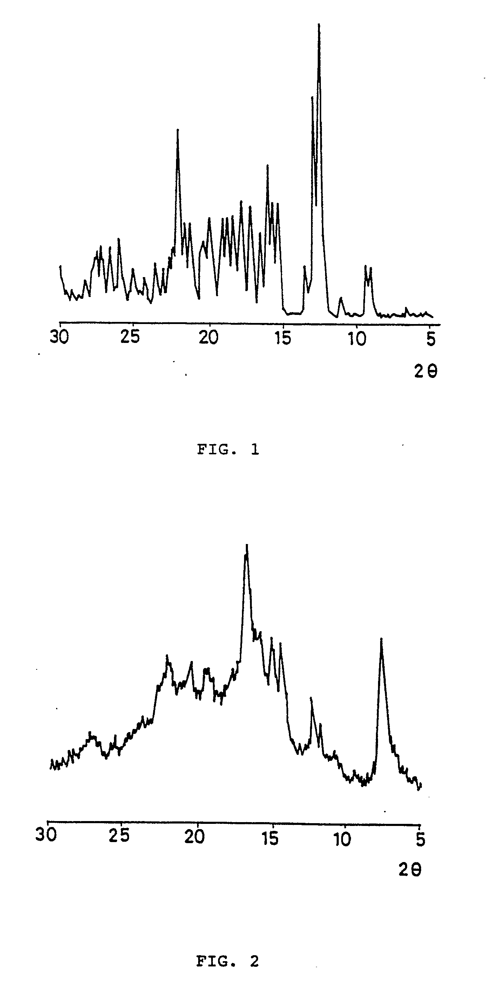

To a suspension of 50 g of β-cyclodextrin (hereinafter abbreviated as "β-CD") in 200 ml of water, was

3

added 5.7 g of α-keto-β-methylvaleric acid (hereinafter abbreviated as "KMV"). After the mixture was stirred at normal temperature for one hour, the crystals were taken out and vaccum-dried overnight to give 47.7 g of KMV/β-CD clathrate compound. The crystals thus obtained were completely free of the disagreeable or inherent to keto-acids,exhibiting slight fruit-like sweetness. The crystals were stable at least for 6 months during storage at room temperature. The analytical values of the crystals are shown in Table 1, and the results of powder X-ray diffraction are shown in Figure 1.

Example 2

To a suspension of 50 g of γ-cyclodextrin (hereinafter abbreviated as "γ-CD") in 150 ml of water, was added 6.7 g of α-keto-isocaproic acid (hereinafter abbreviated as "KIC"). The mixture was treated in the same manner as in example 1 to give 35.7 g of KIC/γ-CD clathrate compound. The crystals thus obtained were also completely free of the disagreeable odor inherent to keto-acids, exhibiting slight fruit-like sweetness. The crystals were stable at least for 6 months during storage at room temperature. The analytical values of the crystals are shown in Table 1, and the results of powder X-ray diffraction are shown in Figure 2.

Example 3

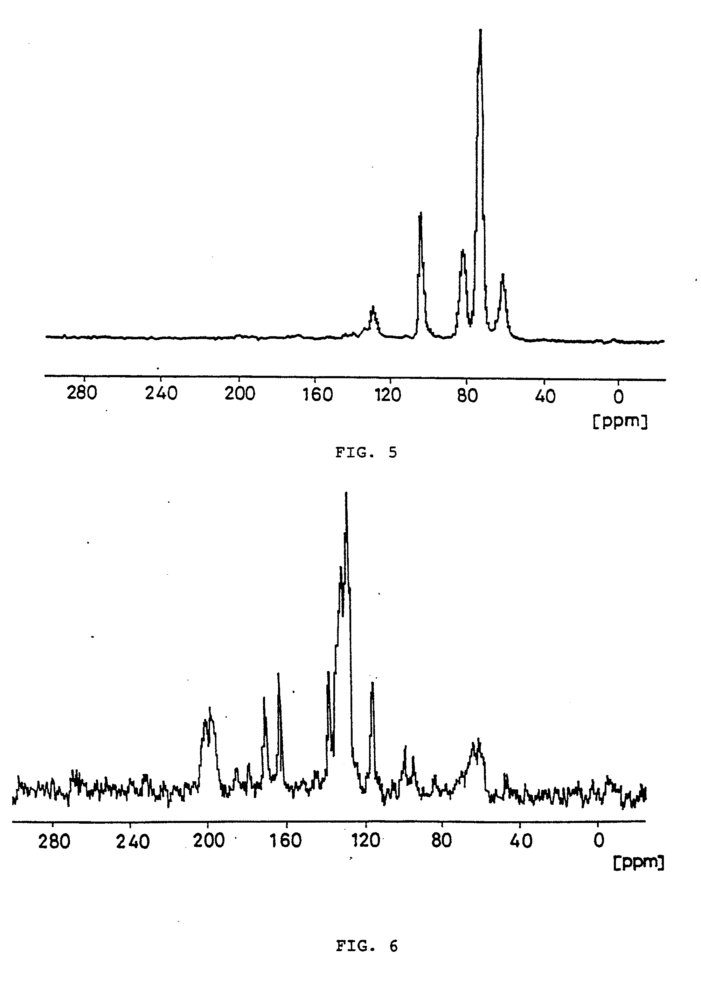

To a suspension of 50 g of β-CD in 150 ml of water, was added 6.4 g of β-phenylpyruvic acid (hereinafter abbreviated as "PPA"). The mixture was treated in the same manner as in Example 1 to give 30.0 g of PPA/β-CD clathrate compound. The crystals were stable at least for 6 months during storage at room temperature. The analytical values of the crystals are shown in Table 1, and the NMR chart as the result of solid NMR spectroscopy is shown in Figure 5.

Example 4

To a suspension of 50 g of β-CD in 200 ml of water, was added 9.0 g of indole-3-pyruvic acid (hereinafter abbreviated as "Ipa"). The mixture was treated in the same manner as in Example 1 to give 41.0 g of Ipa/β-CD clathrate compound. The analytical values of the crystals thus obtained are shown in Table 1. The crystals were odorless and stable over one year or more.

Example 5

To a suspension of 50 g of α-cyclodextrin (hereinafter abbreviated as "α-CD") in 180 ml of water, was added 5.0 g of pyruvic acid (hereinafter abbreviated as "Pyr"). The mixture was treated in the same manner as in Example 1 to give 25 g of Pyr/α-CD clathrate compound. The crystals were odorless white powders and the Pyr content was stable for 3 months or longer. The analytical values of the crystals are shown in Table 1.

The table also lists the analytical values of clathrate compounds prepared from different types of cyclodextrin ("CD" in the table) and keto-acid ("KA" in the table) in the same manner as in Example 1.

4

TABLE 1

| Analytical Values of Keto-Acid/Cyclodextrin Clathrate Compounds Obtained by Crystallization | | | | | | | |
|---|---|---|---|---|---|---|---|
| | | Contents (wt%) | | | Molar Ratio KA/CD | M.P. ($^\circ$C) | Remarks |
| KA | CD | KA | CD | H$_2$O | | | |
| KMV | $\beta$-CD | 9.8 | 83.2 | 5.4 | 1.0 | 218-223 | Example 1 |
| KIC | $\beta$-CD | 10.0 | 88.0 | 3.1 | 1.0 | 220-225 | |
| | $\gamma$-CD | 14.0 | 87.0 | 4.0 | 1.6 | 215-221 | Example 2 |
| KIV | $\beta$-CD | 7.7 | 86.7 | 6.3 | 0.9 | 215-218 | |
| PPA | $\alpha$-CD | 2.1 | 86.5 | 7.6 | 0.1 | 214-219 | |
| | $\beta$-CD | 10.9 | 87.5 | 5.6 | 0.9 | 220-223 | Example 3 |
| | $\gamma$-CD | 13.9 | 74.0 | 5.1 | 1.5 | 205-213 | |
| Ipa | $\beta$-CD | 10.0 | 80.9 | 7.5 | 0.7 | 264-272 | Example 4 |
| Pyr | $\alpha$-CD | 7.0 | 88.0 | 4.3 | 0.9 | 218-222 | Example 5 |

Example 6

To a suspension of 100 g of $\beta$-CD in 400 ml of water, was added 11.5 g of optically active S-$\alpha$-keto-$\beta$-methylvaleric acid (hereinafter abbreviated as "S-KMV") obtained by enzymatic oxidation of L-isoleucine. After the mixture was stirred at normal temperature for one hour, the crystals were separated and collected, and vaccum-dried overnight to give 47.7 g of S-KMV/$\beta$-CD clathrate compound. The crystals were also free of the disagreeable odor inherent to keto-acids (the optical purity of KMV was 99%). It was allowed to stand at normal temperature for four months, and the change in its optical purity was examined as described below. The clathrate compound was suspended in water. After the suspension was adjusted to pH<1 by addition of hydrochloric acid, S-KMV was extracted with ethyl acetate and isolated by distillation. Measurement of its specific rotation showed no reduction in the optical purity.

Comparative Example 1

To 5.6 g of S-KMV obtained in the same manner as in Example 6 , were added 14.2 g of 44% L-lysine (Lys) solution and 150 ml of ethyl alcohol in this order to give 9.5 g of Lys•KMV salt. The salt was allowed to stand at room temperature for 24 hours and then dissolved in pure water. The aqueous solution was adjusted to pK<1 by addition of hydrochloric acid, and KMV was extracted with ethyl acetate and isolated by distillation. Measurement of its specific rotation showed that its optical purity was lowered to 65%.

Example 7

To a suspension of 10 g of $\gamma$-CD in 50 g of water, was added 3.2 g of KIC. After the mixture had been stirred for one hour, it was freeze-dried. The analytical values of the product thus freeze-dried are shown in Table 2.

Example 8

To a solution of 10 g of branched maltosyl-CD (Isoelete®; product of Ensuiko Co., Ltd.) in 50 ml of water, was added 1.1 g of KMV. After the mixture was stirred for one hour, it was freeze-dried. The analytical values are shown in Table 2.

Table 2 also lists the analytical values of clathrate compounds prepared from different types of cyclodextrin and of keto-acids in the same manner as above.

TABLE 2

| Analytical Values of Keto-Acid/Cyclodextrin Clathrate Compounds Obtained by Freeze-drying | | | | | | | |
|---|---|---|---|---|---|---|---|
| | | Contents (wt%) | | | Molar Ration KA/CD | M.P. ($^\circ$C) | Remarks |
| KA | CD | KA | CD | $H_2O$ | | | |
| KIC | $\alpha$-CD | 13.1 | 77.9 | 3.2 | 1.26 | 225-228 | |
| | $\beta$-CD | 17.8 | 74.6 | 5.4 | 2.1 | 232-238 | |
| | $\gamma$-CD | 23.0 | 73.9 | 5.7 | 3.1 | 234-242 | Example 7 |
| | Isoelete | 8.6 | 87.4 | 2.3 | 0.95 | 208-213 | |
| KMV | $\gamma$-CD | 8.8 | 90.8 | 3.6 | 0.96 | 213-220 | |
| | Isoelete | 9.7 | 89.7 | 2.5 | 1.0 | 211-220 | Example 8 |
| PPA | $\alpha$-CD | 20.5 | 67.3 | 7.6 | 1.8 | 203-211 | |
| Ipa | $\beta$-CD | 18.3 | 69.6 | 1.11 | 1.5 | 266-270 | |

Example 9

To 5.0 g of $\alpha$-CD were added 1.2 g of KMV and 1.5 g of water. The mixture was kneaded in a mortar for three hours and then vaccum-dried at 60 $^\circ$C. The clathrate compound thus obtained contained 18.6% of KMV [KMV/$\alpha$-CD = 1.7 (molar ratio)] and showed a melting point of 225-227 $^\circ$C.

EFFECTS ACHIEVED BY THE INVENTION

As is apparent from the foregoing, this invention markedly improves the properties characteristic to $\alpha$-keto-acids, such as disagreeable odor and taste, and low stability and solubility in water, thereby providing novel keto-acid clathrate compounds which can be satisfactorily used as an active ingredient in nutrient and medical compositions.

**Claims**

1. A clathrate compound comprising an a-keto-acid and a cyclodextrin.

2. A clathrate compound according to claim 1, wherein the molar ratio of said $\alpha$-keto-acid to cyclodextrin is in the range from 0.5:1 to 5:1.

3. A clathrate compound according to claim 1 or 2, wherein said cyclodextrin is $\alpha$-, $\beta$- or $\gamma$-cyclodextrin or a mixture of two or all of them.

4. A clathrate compound according to any of the claims 1 to 3, wherein at least a part of the cyclodextrin is mono- or poly-branched cyclodextrin.

5. A clathrate compound according to any of the claims 1 to 4, wherein at least a part of the cyclodextrin is methylated cyclodextrin.

6. A clathrate compound according to any of the claims 1 to 5, wherein said $\alpha$-keto-acid is at least one member of keto-analogues of essential and quasi-essential amino acids.

7. A clathrate compound according to any of the claims 1 to 6, wherein said $\alpha$-keto-acid is S-$\alpha$-keto-$\beta$-methyl valeric acid.

8. A clathrate compound according to any of the claims 1 to 6, wherein said $\alpha$-keto-acid is pyruvic acid.

9. A drug for the treatment and prevention of renal disorders, comprising a clathrate compound according to any of the claims 1 to 8.

10. A drug according to claim 9, which comprises a neutralizer for the clathrate compound.

Claims for the contracting state ES

EP 0 406 811 A2

1. A process for producing a clathrate compound of an $\alpha$-keto-acid and a cyclodextrin, which comprises reacting an $\alpha$-keto acid with a cyclodextrin.

2. A process according to claim 1, wherein the molar ratio of said $\alpha$-keto-acid to cyclodextrin is in the range from 0.5:1 to 5:1.

3. A process according to claim 1 or 2, wherein said cyclodextrin is $\alpha$-, $\beta$- or $\gamma$-cyclodextrin or a mixture of two or all of them.

4. A process according to any of the claims 1 to 3, wherein at least a part of the cyclodextrin is mono- or polybranched cyclodextrin.

5. A process according to any of the claims 1 to 4, wherein at least a part of the cyclodextrin is methylated cyclodextrin.

6. A process according to any of the claims 1 to 5, wherein said $\alpha$-keto-acid is at least one member of keto-analogues of essential and quasi-essential amino acids.

7. A process according to any of the claims 1 to 6, wherein said $\alpha$-keto-acid is S-$\alpha$-keto-$\beta$-methyl valeric acid.

8. A process according to any of the claims 1 to 6, wherein said $\alpha$-keto-acid is pyruvic acid.

9. The use of a clathrate compound producible according to any of the claims 1 to 8 for preparing a drug for the treatment and prevention of renal disorders.

10. The use according to claim 9, wherein a neutralizer for the clathrate compound is added.

7

FIG. 1

FIG. 2

FIG. 3

FIG. 4

9

FIG. 5

FIG. 6

FIG. 7